# EUROPEAN PATENT SPECIFICATION

(11) **EP 0 660 718 B1**
(45) Date of publication and mention of the grant of the patent: **08.12.1999**
(21) Application number: 93921556.2
(22) Date of filing: 14.09.1993
(51) Int. Cl.: A61K 31/56, A61K 33/10, A61K 31/59

(54) **METHOD OF TREATING PREMENSTRUAL SYNDROME SYMPTOMATOLOGY WITH VITAMIN D OR VITAMIN D AND CALCIUM**
VERFAHREN ZUR BEHANDLUNG DES SYMPTOMENKOMPLEXES DES PRÄMENSTRUELLEN SYNDROMS MIT VITAMIN D ODER VITAMIN D UND CALCIUM
PROCEDE DE TRAITEMENT DE LA SYMPTOMATOLOGIE DU SYNDROME PREMENSTRUEL PAR LA VITAMINE D OU LA VITAMINE D ET LE CALCIUM COMBINES

(30) Priority: 15.09.1992 US 945319; 10.05.1993 US 59682
(43) Date of publication of application: 05.07.1995
(73) Proprietor: THYS-JACOBS, Susan, Larchmont, NY 10538 (US)
(72) Inventor: THYS-JACOBS, Susan, Larchmont, NY 10538 (US)
(74) Representative: MacDougall, Donald Carmichael
(86) International application number: PCT/US93/08653
(87) International publication number: WO 94/06435

(56) References cited:
- GB-A- 2 169 202
- GB-A- 2 254 556
- US-A- 4 225 596
- J. CLIN. ENDOCRINOL. METAB., vol. 50, no. 2, 1980, pages 377-379, XP000615935 BARAN ET AL.: "Effect of the Menstrual Cycle on Calcium-regulating Hormones in the Normal Young Woman"
- J. GYNAECOL. ENDOCR., vol. 1, no. 1-2, 1985, pages 52-57, XP000615939 D'ANGELO: "Calciotropic hormones and sex steroids in the early post-menopause: correlation with hydro-electrolyte and mineral metabolism."

## Description

This invention is directed to use of an effective dose of vitamin D in the manufacture of a medicament for treating premenstrual syndrome (PMS).

This invention also relates to use of a therapeutically effective amount of a combination of calcium and vitamin D in the manufacture of a medicament for reducing or relieving symptoms associated with premenstrual syndrome ("PMS").

### BACKGROUND OF THE ART

PMS is characterized by the cyclic recurrence of a variety of emotional and physical symptoms that occur before menses and subside with the onset of menstruation. The temporal occurrence of these symptoms during the luteal phase, rather than their nature, seems to define this phenomenon. However, the precise pathophysiology remains conjectural and obscure.

Symptoms generally experienced by women with PMS without limitation include (1) somatic symptoms such as abdominal cramps, headaches including vascular headaches such as migraine headaches, breast fullness and tenderness, back pain and bloating and (2) psychological symptoms such as depression, irritability and anxiety. The above symptoms do not occur solely in women with PMS. However, it has been estimated that as much as 90% of all premenopausal women exhibit some degree of symptoms such as those above related to PMS, ranging from mild to incapacitating. It has been estimated that about 7 million women suffer severe and incapacitating symptoms related to PMS.

U.S. Patent No. 4,946,679 of Thys-Jacobs ("Thys-Jacobs") disclosed a method for treating premenstrual syndrome ("PMS") by administering to an individual in need of treatment an effective dose of calcium.

Thys-Jacobs et al. disclosed in an article entitled "Calcium Supplementation in Premenstrual Syndrome. A Randomized Crossover Trial" in J. Gen. Int. Med., 1989:4:183F a 50% reduction in PMS symptomatology with daily administration of elemental calcium in a dose of 1000 mg for three months.

Chuong et al., in an abstract entitled "Calcium Levels in Premenstrual Syndrome" presented at the American Fertility Annual Meeting in 1991 showed that women with PMS had significantly lower calcium levels during the luteal phase of the menstrual cycle as compared with asymptomatic controls and also showed that women with PMS had significantly lower calcium levels during the luteal phase of the menstrual cycle as compared to the follicular phase of the menstrual cycle.

It is believed that calcium plays a role in the release of neurotransmitters, endocrine and exocrine products, in the contraction of skeletal and smooth muscle, and metabolism. Menstruation is related to ovarian and pituitary secretory function. Thys-Jacobs disclosed that calcium, coupled with ovarian linked hormones, may modulate the intrinsic feedback mechanism that translates physiological neuroendocrine and hormonal messages into behavioral and somatic changes.

However, there still exists a need for therapy that provides further reduction or relief of symptoms associated with PMS, especially in particularly persistent cases.

GB 2 169 202 describes compositions for providing relief from menstrual stresses. The compositions comprise calcium carbonate, magnesium hydroxide, vitamin C as calcium ascorbate, pantothenic acid, vitamin B₆, vitamin D and vitamin E.

GB 2 254 556 discloses compositions for administration to adolescent girls to prevent the onset of premenstrual syndrome. The compositions comprise linoleic acid and one or more of a calcium source, an iron source, and vitamin B₆. The compositions may contain other components such as magnesium, vitamins A, B1, B2, B3, B12, folic acid, C, D and E.

### SUMMARY OF THIS INVENTION

An object of this invention is to reduce or relieve symptoms associated with PMS in an individual exhibiting symptoms of PMS, especially in those patients who do not demonstrate improvement when treated with calcium alone.

A further object of this invention is to reduce or relieve symptoms associated with PMS in an individual both exhibiting symptoms of pMS and demonstrating low vitamin D levels.

The present invention provides use of an effective dose of vitamin D in the manufacture of a medicament for significantly reducing the symptoms of premenstrual syndrome.

The present invention also provides use of a combination of calcium and vitamin D in the manufacture of a medicament for at least reducing the symptoms associated with premenstrual syndrome symptomatology.

### BRIEF DESCRIPTION OF THE DRAWINGS

**FIG. 1** shows a comparison of vitamin D levels in 26 women exhibiting symptomatology associated with PMS and in 20 asymptomatic controls.
**FIG. 2** shows a comparison of bone mineral densities at the lumbar spine L2-4 in 26 women exhibiting symptomatology associated with PMS and in 20 asymptomatic controls.

### DETAILS DESCRIPTION OF THE INVENTION

Vitamin D has many known functions. For example, in the intestine, 1,25 dihydroxyvitamin D ("1,25(OH)₂D") increases the absorption rate of calcium, phosphorous and magnesium throughout the small and large bowels. 1,25(OH)₂D also increases the transport of calcium from the extracellular space, and it can mobilize intracellular calcium concentrations from intracellular calcium pools. Further, vitamin D induces the synthesis of proteins in the intestinal epithelial brush border epithelial cells. Receptors for 1,25(OH)₂D are present in many organs and tissues. They have been found in the intestine, kidneys, bone, skin, breast, pituitary gland, parathyroid glands, beta cells of the pancreatic islets, gonads, brain, skeletal muscle, circulating monocytes and lymphocytes.

Vitamin D deficiency is known to impair insulin secretion and glucose tolerance, and to impair normal cellular differentiation. Furthermore, vitamin D deficiency has been associated with chronic muscle weakness and myopathy.

Applicant has now found a relationship between vitamin D deficiency and women exhibiting PMS symptomatology.

The medicament of the present invention treats PMS by administering to an individual in need of treatment an effective dose of vitamin D so that the symptoms of PMS are significantly reduced or relieved. The vitamin D may be administered in the form of vitamin D2 (ergocalciferol), vitamin D3 (cholecalciferol), calcifediol [25(OH)], and the like. Preferably, the dosage of vitamin D administered is in the range of from 200 to 2000 IU per day, most preferably as vitamin D2 or D3. For example, a recommended daily dosage of vitamin D2 or vitamin D3 is 400 IU. For individuals exhibiting PMS symptomatology and a marked vitamin D deficiency, the preferred dosage is between 50,000 to 200,000 IU weekly for 2-3 months. When the treatment comprises administering calcifediol, the preferred dosage of calcifediol is between 800 to 8000 IU (20 to 200 ug) daily, preferably for between 2 to 4 weeks. Lower doses may be used to prevent recurrent symptoms. Preferably, the dosage is administered orally, most preferably in the form of a tablet. The vitamin may also be administered intramuscularly such as by an injection in an oil base. For example, vitamin D2 is preferably administered intramuscularly in an oil base in a dosage of 500,000 IU or 12.5 mg every 2-3 months.

The medicament of the present invention also treats individuals exhibiting symptoms associated with PMS by the administration of a therapeutically effective amount of a combination of calcium and vitamin D. Preferably, the dosage of elemental calcium administered is in the range of from about 1000 mg to about 2000 mg per day. Preferably, the dosage of vitamin D administered is in the range of from about 400 to about 2000 IU per day. Preferably, the dosage of vitamin D elevates 25 hydroxyvitamin D levels to levels greater than 30-40 ng/ml. The calcium and vitamin D may be administered concurrently such as, for example, by administration of a tablet, a capsule, a powder, liquid, candy or mint, cookie or food additive containing the desired dosages of the calcium and the vitamin D. Preferably, the combination is administered orally in the form of a tablet. Calcium may be administered in the form of calcium carbonate, calcium gluconate, calcium citrate, calcium phosphate, calcium chloride, calcium stearate or calcium acetate, and preferably in the form of calcium carbonate. Vitamin D may be administered as at least one of vitamin D₂ (ergocalciferol), vitamin D₃ (cholecalciferol) or 25 hydroxyvitamin D (calcidiol or calcifediol). The dose can be taken as a single daily combination dose or in split doses of smaller concentrations in adequate levels for prevention of PMS symptoms. Examples of combinations for single doses are as follows:

| Elemental calcium | Vitamin D₂ or D₃ |
|---|---|
| 1000 mg | 400 IU |
| 1000 mg | 600 IU |
| 1000 mg | 800 IU |
| 1200 mg | 400 IU |
| 1200 mg | 600 IU |
| 1200 mg | 800 IU |
| 1200 mg | 1000 IU |
| 1200 mg | 1200 IU |
| 1500 mg | 400 IU |
| 1500 mg | 300 IU |
| 1500 mg | 800 IU |
| 1500 mg | 1000 IU |
| 1500 mg | 1200 IU |
| 1500 mg | 2000 IU |
| 1600 mg | 1600 IU |
| 1800 mg | 1800 IU |

Examples of smaller concentration embodiments to be administered at least 2 to 3 times daily are as follows:

| Elemental calcium | Vitamin D₂ or D₃ |
|---|---|
| 300 mg | 200 IU |
| 300 mg | 250 IU |
| 500 mg | 200 IU |
| 500 mg | 300 IU |
| 500 mg | 400 IU |
| 600 mg | 300 IU |
| 600 mg | 400 IU |
| 600 mg | 500 IU |
| 600 mg | 600 IU |
| 700 mg | 700 IU |
| 800 mg | 400 IU |
| 800 mg | 500 IU |
| 800 mg | 800 IU |
| 1000 mg | 1000 IU |
| 2000 mg | 2000 IU |

Vitamin D and the above combination of calcium and vitamin D are effective for reducing or relieving symptoms associated with PMS, which include somatic symptoms such as without limitation headaches, especially vascular headaches such as migraine headaches, tenderness and swelling of the breasts, abdominal bloating, abdominal cramping, generalized aches and pains, lower backache, fatigue, increased/decreased appetite, craving for sweet/salt, swelling or edema of extremities and insomnia and which include psychological symptoms such as mood swings, depression, tension, anxiety, anger and crying spells.

### EXAMPLES

The foregoing and other objects, features, aspects and advantages of the present invention will become more apparent from the following detailed non-limiting examples of the present invention.

### Example 1

### Applicant's Research study Indicating That PMS Is Associated With Low Vitamin D Levels

The study herein described measures several parameters in women with PMS symptomatology and compares these results with premenopausal asymptomatic controls. The study was conducted in the Clinical Research Center and outpatient department at Mount Sinai Medical Center.

### 1.1 Enrollment

Subjects ("PMS group") and controls were recruited by advertisement or privately referred. One hundred thirty-eight women were initially screened for the trial. Those women who reported having PMS were screened for the PMS group; those who presented without symptoms of PMS were screened for the control group.

Participants for the PMS group were selected if: 1) they met preset criteria based on a prospective and consecutive 2 month diary: a pattern of clinically significant emotional and somatic symptoms that occur during the last week of the luteal phase and remit within a few days after the onset of the follicular phase, 2) they demonstrated a mean symptom intensity change of at least 50% in the luteal phase compared to the intermenstrual phase and 3) they had no history of metabolic bone disease or condition known to affect the bones. Controls were selected if they did not manifest symptoms of PMS based on the 2 month prospective diary.

The prospective assessment of daily symptoms for two menstrual cycles was obtained by means of a questionnaire which contained sixteen symptoms rated as absent, mild, moderate or severe and scored from 0 to 3. These sixteen symptoms were: nervousness, irritability, crying, mood swings, depression, increase or decrease in sleep, fatigue, difficulty in concentration, violent tendencies, abdominal bloating, headache, breast fullness, change in appetite, abdominal cramps, back pain, and craving for chocolate or salt.

The following were exclusion criteria for both groups: (1) history of amenorrhea, (2) menstrual cycle irregularity, (3) anorexia nervosa, (4) hyperparathyroidism, hyperthyroidism or hypothyroidism on dosages of levothyroxine greater than .05 mg per day, (5) major psychiatric disorder or active depression, (6) history of smoking more than one pack per day, (7) pregnancy, (8) perimenopause or menopause, history of endometriosis, mental retardation, (9) use of thiazide diuretics or anticonvulsants, (10) history of corticosteroid use, or (11) metabolic bone disease. Women with psychiatric disorders, severe anxiety and depression were eliminated as determined by an extensive psychological screening which included the Beck Inventory Depression Scale, the Spielberg State Trait Anxiety Inventory, and the Premenstrual Assessment Form-past cycle version of Halbreich, Enicott and Schacht. Clinical evaluation also involved a detailed medical, menstrual and gynecological history and a physical examination.

Final selection of the PMS group and controls was achieved by consensus among the internist, the psychiatrist, and the clinical social worker upon thorough review of the PMS diary, medical history and the psychological evaluation of each woman. All selected participants were caucasians, who gave informed consent in accordance with the policy established by Mount Sinai Medical Center's Institutional Review Board. 26 women were selected for the PMS group and 20 for the controls. 92 women were excluded based either on the above-described inclusion criteria or noncompliance with the initial intake evaluation. The majority of the women were working women in professions such as law, medicine, nursing, social work and teaching. Most held full-time positions in their field. All were white, middle to upper class women residing in the New York metropolitan area or suburbs, who were financially self-sufficient. As a group, they were knowledgeable of the clinical presentation of PMS and were well-informed from the media, self-help books and their own physicians about the current and available treatments for PMS. The majority of the women with PMS had attempted at least one form of recommended therapy with unsuccessful alleviation of their symptoms.

### 1.2 Study

### a. Clinical and biochemical characteristics of the women in the PMS and control groups

Serum follicular stimulating hormone, luteinizing hormone, progesterone, magnesium, calcium, albumin, alkaline phosphatase, a complete blood count, and a 24 hour urine calcium excretion were obtained in all women during the luteal phase and measured by the clinical laboratories at Mount Sinai Medical Center. Both serum and urine calcium were measured using a colorimetric method on Beckman CX3 instruments. Calciotropic hormone samplings of 1,25 dihydroxyvitamin D [1,25(OH)₂D], 25 hydroxyvitamin D [250HD], intact parathyroid hormone (iPTH) were performed by USCF Hormone Reference Laboratory. Serum progesterone was measured during the last seven days of the menstrual cycle to confirm ovulation.

The test results are shown in Table 1.

**TABLE 1**

| CLINICAL AND BIOCHEMICAL CHARACTERISTICS OF THE WOMEN IN THE PMS AND CONTROL GROUPS | | |
|---|---|---|
| | PMS GROUP | CONTROL |
| Number of Subjects | 26 | 20 |
| Mean Age in years | 34.6±5.7 | 34.3±1.0 |
| Mean Age at Menarche | 12.4±1.5 | 12.3±1.0 |
| Number of Term Pregnancies | 0.46±71 | 0.45±89 |
| History of Smoking | 59.7% | 40.0% |
| Exercising regularly | 61.5% | 70.0% |
| Weight (kilograms) | 58.5±7.7 | 59.9±5.9 |
| Height (centimeters) | 162.6±5.0 | 162.6±5.0 |
| Body Mass Index (kg/m2) | 22.3±3.0 | 22.8±1.8 |
| Alkaline Phosphatase (units/liter) | 61.5+27.6 | 56.3±16.2 |
| Serum Calcium (mmol/liter) | 2.4±0.1* | 2.3±0.1 |
| Serum iPTH (pg/ml) | 21.1±11.0 | 25.1±12.41 |
| Serum 1,25(OH)2D (pg/ml) | 37.81±8.1 | 37.2±6.1 |
| Serum 250HD (ng/ml) | 19.5±7.5** | 25.3±8.3 |
| % Calcium Excretion | 1.4±0.5 | 1.2±0.7 |
| Serum Albumin | 4.5±0.3 | 4.4±0.7 |
| Serum Magnesium | 2.06±0.23 | 1.96±0.13 |
| Serum Creatinine | 0.87±0.2 | 0.88±0.2 |
| Serum Cholesterol | 183.8±27.2* | 215.5±19.0 |
| Data are expressed as means ± standard deviations or percentages for yes/no answers. | | |

| | | |
|---|---|---|
| *p value <0.05. | | |
| **P value <0.02. | | |

### b. Dietary characteristics of the women in the PMS and control groups

All participants, under prior instruction and supervision by a trained clinical research center's dietician, kept a detailed record of food intake for 3 days. The type of food consumed and the serving size of each portion for every meal and snack were reported. Portion sizes were estimated by the use of common kitchen utility measuring containers. Mean calcium intakes were determined for each subject by averaging the 3 daily intakes.

The test results are shown in Table 2.

**TABLE 2**

| DIETARY CHARACTERISTICS OF THE WOMEN IN THE PMS AND CONTROL GROUPS | | |
|---|---|---|
| | PMS GROUP | CONTROL |
| Number of Subjects | 26 | 20 |
| Calcium (mg) | 726.5±329.7 | 736.0±164.1 |
| Carbohydrate (gm) | 184.7±56.7 | 169.7±40.3 |
| Protein (gm) | 64.2±16.1 | 62.8±12.1 |
| Fat (gm) | 57.8±14.5 | 47.2±18.9 |
| Caffeine (mg) | 270.7±212.8 | 196.8±152.1 |
| Cholesterol (mg) | 204.9±67.1 | 185.6±107.2 |
| Calories | 1535.6±355.6 | 1401.3±254.4 |
| Potassium (mg) | 2006.3±522.1 | 2164.2±552.5 |
| Sodium (mg) | 1679.2±491.6 | 1887.4±505.5 |
| Phosphorus (mg) | 841.5±257.5* | 1022.8+171.2 |
| Magnesium (mg) | 180.7±72.7 | 207.3±48.5 |
| Zinc (mg) | 5.7±2.1 | 7.9±4.3 |

| | | |
|---|---|---|
| *P value <0.05 | | |

### c. Daily symptoms in the PMS and control groups

A total daily symptom rating score was derived by summing the 16 individual symptom ratings. Mean symptom scores for the different phases of the menstrual cycle were derived from the daily symptom ratings as follows:
- Luteal mean:: the average score of total symptoms for all cycles seven days prior to onset of the menstrual period
- Menstrual mean:: the average score of total symptoms for all cycles on the days of menstruation
- Maximum Luteal:: the highest luteal average score for a single cycle
- Maximum Menstrual:: the highest menstrual average score for a single cycle
- Intermenstrual mean:: the mean score of the total symptom scores during the seven days following the menstrual period for all cycles

The test results are shown in Table 3.

**TABLE 3**

| MEAN SYMPTOM SCORES FOR THE LUTEAL AND MENSTRUAL PHASES IN THE PMS AND CONTROL GROUPS | | | | |
|---|---|---|---|---|
| MEAN SYMPTOM SCORES | PMS | Control | Rank Sum Z Statistic | Exact p Value |
| Luteal Phase | | | | |
| Luteal Mean | 13.3±7.3 | 1.5±1.4 | 5.618 | 0.0000 |
| Maximum Luteal | 16.3±9.6 | 2.0±1.6 | 5.652 | 0.0000 |

| Menstrual Phase | | | | |
|---|---|---|---|---|
| Menstrual Mean | 9.5±6.4 | 1.9±1.7 | 5.163 | 0.0000 |
| Maximum Menstrual | 12.1±7.7 | 2.5±2.0 | 5.231 | 0.0000 |
| Intermenstrual Mean | 2.6±2.5 | 0.5±0.8 | 3.915 | 0.0000 |
| Data are expressed as means ± standard deviations. | | | | |

### d. Bone mineral density

Bone mineral content of the lumbar spine and the proximal femur of all the women was determined by dual photon absortiometry using the model DP3 system (Lunar Corp., Madison WI). Transmission scanning used a Gadolinium-153 source with two separate energy windows (44 and 100 KeV). The separate energies permitted the determination of the bone mineral content independent of the amount of soft tissue present. Bone mineral content (BMC) was measured in grams. Bone mineral density ("BMD") was derived by dividing the BMC by the area of the scanned bone.

The primary region of interest in the lumbar spine was L2 through L4. Vertebral BMD measurements were also determined for L1-2. For vertebral scanning of the lumbar spine L1-L4, the body weight and height were measured. Subjects adopted the supine position with legs elevated over a cube to minimize lumbar lordosis. The iliac crest was identified by PALPATION and the probe placed in the scanning position at L5. Rectilinear scans (40 scan lines, line thickness 4.5 mm apart with a 2.5 mm/second speed) were performed on each subject.

The right proximal femur was scanned beginning at the symphysis pubis in the supine position, after the lumbar positioning cube was removed. An angular brace was strapped to the right ankle, and the right leg rotated as far as possible for best visualization of the femur. Bone mineral measurements were determined for the femoral neck, Ward's triangle and the greater trochanter.

All subjects were positioned and data analyzed by a single trained technician. The coefficient of variation and the long term reproducibility of these procedures in our center is ± 2%.

The test results are shown in Table 4.

**TABLE 4**

| BONE MINERAL DENSITY MEANS | | | | |
|---|---|---|---|---|
| | PMS | Control | Rank Sum Z Statistic | Exact P Value |
| Spine | | | | |
| L2-4 | 1.18±1.1 | 1.28±.11 | 3.092 | 0.0016 |
| L1-4 | 1.14+1.0 | 1.24±.11 | 2.892 | 0.0032 |
| L1-2 | 1.17±1.0 | 1.26±.11 | 2.947 | 0.0027 |

| Femur | | | | |
|---|---|---|---|---|
| Femoral neck | 0.94±.10 | 0.97±.14 | 0.798 | 0.4316 |
| Wards triangle | 0.84±.10 | 0.91±0.16 | 1.996 | 0.0458 |
| Trochanter | 0.76+11 | 0.80±.10 | 1.486 | 0.1398 |
| Data are expressed as means ± standard deviations | | | | |

### e. Correlation between mean symptom scores and selected BMD and calciotropic hormone levels

Blood samples for measurement of parathyroid hormone, 1,25 dihydroxyvitamin D, and 25 hydroxyvitamin D were collected in serum separator tubes, allowed to clot at room temperature for 30 minutes and immediately centrifuged for separation of the serum. Plastic pipettes were used to transfer the serum to plastic sample vials that were immediately frozen at -70 degrees centigrade.

PTH was measured with an assay previously reported by Nussbaum with minor modifications. This assay is a two site immunoradiometric assay employing two polyclonal antibodies for the measurement of biologically active PTH (1-84). Only the intact PTH fragments form the sandwich complex necessary for measurement of the radiolabelled antibodies to human PTH. One is specific for the PTH midregion or the C terminal (39-84) and is immobilized by adsorption onto 8 mm diameter polystyrene beads, and the other is radiolabelled for the N terminal region of PTH 1-34. The serum samples were assayed in duplicate. The intraassay and interassay coefficients of variation were 5.3% and 8.5% respectively. The normal range is 10 to 60 pg/ml. The assay is linear over the range of 10 pg/ml to 1650 pg/ml.

The 1,25 dihydroxyvitamin D samples were extracted by an acetonitrile method as described by Reinhards to remove proteins and lipids. Samples were purified on C-18 and Silica Sep-pak cartridges in a consecutive manner. A recovery sample was pipetted to measure the extraction efficiency of each sample. The purified extracts were then assayed in duplicate in a radio-receptor assay. The assay utilizes calf thymus and binding protein which contains receptors which are specific for 1,25 dihydroxyvitamin D, permitting quantitative measurements in the range of 1.5 to 40 picograms per tube. The intraassay and interassay variations are 6.6% and 12.4% respectively.

The 25 hydroxyvitamin D samples were extracted using methanol to remove proteins and lipids. Extracts were then chromatographed on a Sep-pak cartridge in order to separate the 25 hydroxyvitamin D fraction from other vitamin derivatives. Recovery of 25 hydroxyvitamin D in each sample was monitored through the extraction and chromatography steps. The chromatographed extracts were assayed in duplicate in a competitive protein binding assay. Two dilutions were used to assure parallelism with standards. The assay utilized a naturally occurring vitamin D binding protein found in rat serum. The assay permitted quantitative measurements in the range of 1.5 to 40 picograms per tube. The intraassay and interassay variations for this assay were 10% and 15% respectively.

Pearson product correlation coefficients with 95% confidence intervals were calculated to assess the relationship between both luteal and menstrual symptomatology and various factors such as age, weight, BMD and calciotropic hormones.

The test results are shown in Table 5.

**TABLE 5**

| CORRELATION COEFFICIENTS BETWEEN MEAN SYMPTOM SCORES AND SELECTED BMD AND CALCIOTROPHIC HORMONE LEVELS IN THE TOTAL STUDY SAMPLE | | | | |
|---|---|---|---|---|
| | Luteal Mean | Max Luteal** | Menstrual Mean | Max Menstrual*** |
| Serum Calcium | -0.01 (-0.30, 029) | 0.01 (-0.29, 0.30) | -0.13 (-0.41, 0.17) | -0.12 (-0.40, 0.18) |
| iPTH | -0.30 (-.54, -0.01)* | -0.30 (-0.54, -0.01)* | -0.23 (-0.49, 0.06) | -0.23 (-0.49, 0.06) |
| 1.25(OH)D | 0.14 (-0.16, 0.41) | 0.19 (-0.11, 0.46) | 0.03 (-0.26, 0.32) | 0.04 (-0.25, 0.33) |
| 250HD | -0.19 (-0.46, 0.10) | -0.17 (-0.44, 0.13) | -0.19 (-.46, 0.11) | -0.20 (-.47, 0.09) |
| Dietary Calcium | -0.20 (-0.50, 0.14) | -0.28 (-0.56, 0.06) | -0.33 (-0.60, 0.01) | -0.35 (-0.61, -0.01)* |
| Spinal L2-4 | -0.38 (-0.61, -0.11)* | -0.36 (-0.59, -0.07)* | -0.25 (-0.51, 0.04)* | -0.29 (-0.53, 0.00)** |
| 95% confidence limits for the correlation coefficients are given within parenthesis. | | | | |

| | | | | |
|---|---|---|---|---|
| *P value <0.05. | | | | |
| **Max Luteal is the abbreviated form of Maximum Luteal. | | | | |
| ***Max Menstrual is the abbreviated form of Maximum Menstrual. | | | | |

### 1.3 Discussion of Results

### a. Biochemical and calciotropic hormone values

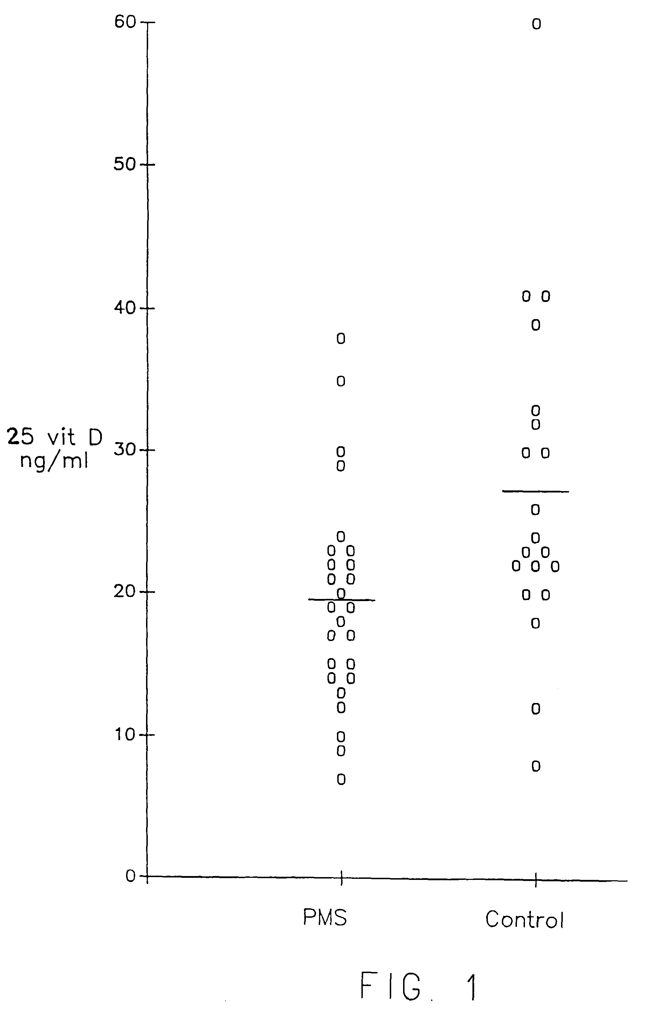

As shown in Table 1, biochemical and calciotropic hormone values were normal in both groups with the exception of 25OHD which were below the normal range of 10 ng/ml in two women with PMS and one control. However, even within normal biochemical parameters, there were differences between the controls and women with PMS. Compared with normal controls, women with PMS had significantly lower 25OHD levels (19.5±7.5 vs. 25.3±8.3 ng/ml; t=2.46, df=44, P=.018 [Figure 1]) and higher serum calcium levels (2.4±0.1 vs. 2.3±0.1 mmol/liter; t=2.03, df=43, P=0.049). Serum cholesterol levels were significantly lower in the PMS group than in the controls (183.8±27.2 vs. 215.5±19.0; t=2.19, df=20, P=0.04). There were no differences between the two groups in the mean values iPTH, 1,25(OH)₂D, % calcium excretion, magnesium or albumin.

### b. Dietary comparisons

Table 2 shows dietary comparisons between the two groups. Phosphorous intake was significantly lower in the PMS group than in the controls (841.5±257.5 vs. 1022.8±171.2 mg; t=2.36, df=33, P=0.024). Mean 24 hour dietary calcium intakes were 726.5 mg. for the PMS group and 736.0 mg for controls. This did not prove significantly different. There were no differences in mean dietary intake of fat, carbohydrates, protein, cholesterol or calories.

### c. Mean symptom scores for the luteal, menstrual and intermenstrual phases

Table 3 shows the mean symptom scores for the luteal, menstrual and intermenstrual phases of the menstrual cycle. As expected, the PMS group had significantly higher luteal symptoms rating scores (luteal mean and maximum luteal) than controls (P=0.000). Menstrual phase symptom rating scores (menstrual mean and maximum menstrual) were similarly higher in the PMS group compared to control groups (P=0.000). All participants with PMS demonstrated a mean symptom intensity change in the range of 500% in the luteal phase relative to the intermenstrual phase, well above the minimum change of 50% required by the inclusion criteria.

### d. Bone mass measurements

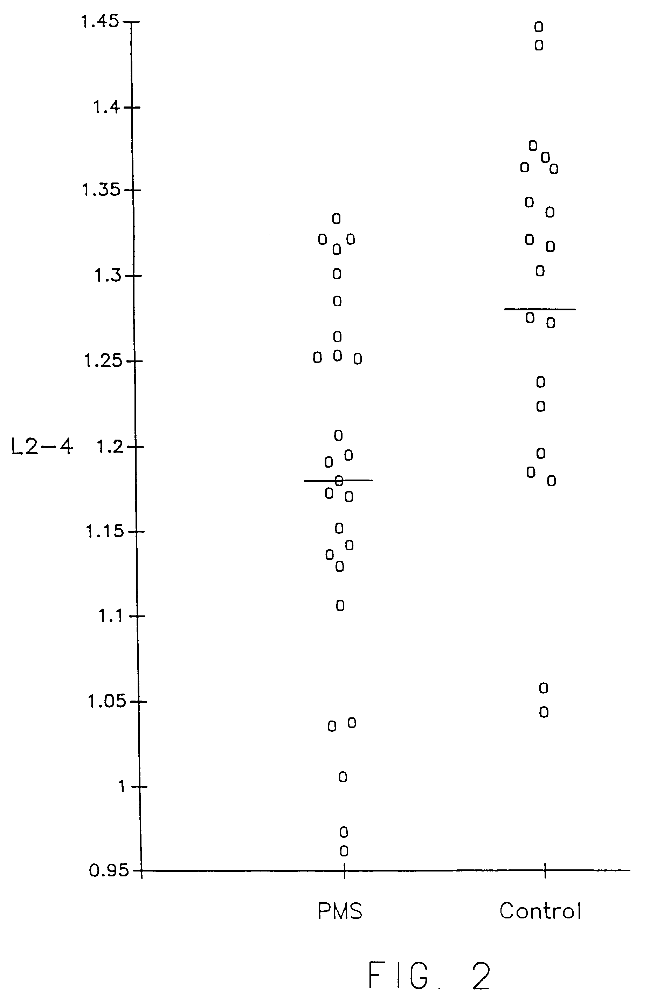

Table 4 shows means and standard deviations on bone mass measurements were performed at the lumbar spine and the proximal femur for women with PMS and controls. The PMS group had reduced bone mass at the L2-4 site compared to controls. The mean ±SD BMD in the PMS group was 1.18±.11 compared to 1.28±.11 among controls (Wilcoxon rank sun Z = 3.09, P=0.0016). A notable downward shift in the range of bone mass measurements at the L2-4 among the PMS participants is illustrated in Figure 2. Bone mass was also reduced in the PMS group compared to the control group in the L1-4 site (1.17±.10 vs 1.24±.11; Wilcoxon rank sum Z= 2.89, P = 0.0032) and in the L1-2 site (1.14±.10 vs 1.24±.11); Wilcoxon rank sum Z = 2.95, P=0.0027). Of the femoral bone mass measurements, the Ward's triangle area was significantly reduced in the PMS group compared to the control group (0.84±.10 vs. 0.91±.16; Wilcoxon rank sum Z = 2.00, P = 0.0458). The groups did not differ in the BMD for the femoral neck or the trochanter.

### e. Correlation between mean symptom scores and BMD and calciotropic hormone measurements

Table 5 shows the correlation between mean symptom scores and BMD and calciotropic hormone measurements in the total sample. The same correlation was performed for the PMS group. Parathyroid hormone was negatively correlated with luteal symptomatology in the total group (r=-0.30; 95% CI -054, -0.01). Dietary calcium negatively correlated with the menstrual mean (r=0.)3;95% CI - 0.60, 0.01) and the maximum menstrual (r=-0.35;95% CI - 0.61, -0.01). The spinal L2-4 BMD correlated -.38 (95% CI -.61 to -.11) with luteal symptom scores, again consistent with the finding of significant differences between groups on the spinal BMD measurements. However, the correlation of L2-4 with menstrual symptom scores was smaller and had a 95% CI which included zero (r=-.25; 95% CI -.51 to .04). Similar patterns can be seen for the L1-4 and L1-2 measurements. To test whether higher luteal symptomatology was associated with lower bone mass, particularly in the spine, correlations between the spinal bone mass measurements and luteal symptom scores in the PMS group were computed. There was a reduction in the correlations when the analysis was limited to the PMS group. The correlation between L2-4 and luteal symptom scores was -.18 (95% CI - .52,.22). The negative correlations between the spinal BMD measurements and luteal symptom severity can thus be attributed mainly to the differences between PMS and control groups in bone mass and not to a linear increase in symptom severity with reduced bone mass.

Consistent with total sample correlations, dietary calcium in the PMS group negatively correlated with menstrual symptom means. Serum calcium was negatively correlated with both luteal and menstrual symptom means. The correlations of luteal symptoms with iPTH seen in the total sample were not detected in the PMS group.

Many women may rate their symptoms differently according to their individual tolerance to pain. To control for this possibility, we used the intermenstrual symptom means as a covariate. The luteal and menstrual symptom means were correlated with the BMD and the calciotrophic hormone measurements controlling for the intermenstrual symptom mean. The results which stand out in these analysis are the positive correlations between 1,25(OH)₂D and luteal symptoms both in the PMS group (r=0.41; 95% CI 0.02,0.69; P=0.04) and the total sample (r=0.29; 95% CI 0.00,0.53; P=0.05).

### 1.4 Results

The data demonstrated a significant difference in vitamin D between the two groups of women with lower levels of 25OHD in the PMS group compared to the controls. The reduced bioavailability of vitamin D may be reflective of inadequate formation of vitamin D from precursor 7-dehydrocholestrol or simply inadequate dietary intake. While two of our PMS participants had abnormally low 25OHD levels, the majority had normal though lower levels compared to controls.

Both the PMS and control participants were white, premenopausal women who were similar in age, body composition and activity with no history of bone disease or menstrual disorders. The mean vertebral bone mineral density value at L2-4 (1.28 g/cm² ±0.11) in our normal controls was comparable to that reported by Mazess et al. for U.S. white women (1.26 g/cm² ±0.13). Although the mean age of our participants with PMS was 34 years, the mean vertebral BMD of these participants at L2-4 (1.18 g/cm² + ±0.11) was equivalent to that of a woman 16 years older based on Mazess' age regression equation predicting spinal density. The standard deviations of 0.11 for the control group's vertebral BMD was comparable to the reported standard deviation of 0.13 for ages 30 to 39. For the femur, our control's standard deviation was 0.10 to 0.16 compared to Mazess' report of 0.12 to 0.14.

Recent evidence in the pattern of bone loss has revealed a significant premenopausal bone loss in the appendicular and axial skeleton. The data shows a significant premenopausal vertebral and femoral bone loss in women with PMS. Other clinical surveys have similarly demonstrated a premenopausal bone loss in these two areas. In 1987, Mazess and colleagues showed premenopausal bone losses in their cross sectional data for the spine and femur with as much as 50% of the 20-25% reduction in spine and femur BMD evident prior to menopause. The longitudinal study by Riggs and colleagues noted a continuous and significant vertebral bone loss in 139 women of 1.32% per year before menopause and 0.97% per year after menopause. Significant bone loss from the axial skeleton was identified before menopause, while cortical bone loss as measured in the midradius remained insignificant.

High dietary calcium intake has been associated with increased serum calcium levels and suppression of parathyroid hormone. In the present study, women with PMS had higher serum calcium concentrations and lower 25OHD levels than controls. This was an unexpected finding. Lower serum concentrations of calcium had been expected, since correlations of mean symptom scores with serum calcium, dietary calcium and iPTH were negative ones, while those of 1,25(OH)₂D were positive. A partial deficiency of vitamin D with low 25OHD levels can result in a secondary hyperparathyroidism with increased levels of 1,25(OH)₂D. This may explain the positive correlations detected in 1,25(OH)₂D levels with mean luteal scores. The secondary hyperparathyroidism that is expected in women with PMS may eventually be blunted from higher serum calcium levels with a resetting of the calcium set point.

The results of the present study indicate that women with PMS have reduced bone mass measurements compared to asymptomatic controls. The precise mechanism of the reduced bone mineral density in women with PMS is not known, although a long term partial vitamin D deficiency may be involved.

### EXAMPLE 2

### Applicant's Research Study Indicating That Many Women With PMS Have A Vitamin D Deficiency And That Symptoms Associated With PMS Can Be Reduced Or Relieved With A Treatment Of A combination Of Vitamin D And calcium

### 2.1 Enrollment of Participants

The study herein described was conducted at Mount Sinai Hospital in New York City. Women working and residing in the New York area with a self-diagnosis of PMS were recruited.

From those women reporting a self-diagnosis of PMS, women were further selected if they fulfilled a strict definition of premenstrual syndrome: Cyclically recurring symptoms during the luteal phase of the menstrual cycle which subside with the onset of menstruation. Determination of recurrence of symptoms was based on a prospective and consecutive two month daily diary. Each woman was asked to complete daily pre-trial self-assessment questionnaires where 17 symptoms were measured and recorded daily over one menstrual cycle. Each was instructed to complete one questionnaire every evening, describing how she felt during the previous 24 hours by recording her level of symptom severity for each of the seventeen symptoms. The 17 symptoms evaluated were: mood swings, depression, tension, anxiety, anger, crying spells, tenderness and swelling of breasts, abdominal bloating, abdominal cramping, generalized aches and pains, low backache, headache, fatigue, increased/decreased appetite, cravings for sweet/salt, swelling/edema of extremities and insomnia. Each symptom was marked daily on a four-point scale (absent, mild, moderate, severe) and subsequently scored from 0 to 3. Women were further selected if their mean symptom scores from the latter seven days of the luteal phase were at least 50% greater than the seven days following the days of menstruation.

Criteria for exclusion from the clinical trial were: (1) history of renal disease, 2) history of primary hyperparathyroidism, (3) history of liver or gastrointestinal disease, (4) history of endometriosis, (5) history of psychosis and (6) active depression.

22 women were finally selected for this study. A preliminary evaluation on each finally selected woman ("patient") included (1) a standardized medical evaluation with a detailed gynecological history as well as a routine physical examination and (2) a determination of complete blood count, electrolytes, alkaline phosphatase, albumin, glucose and urinalysis. All determinations of the above were within normal laboratory limits as set by the laboratory performing the determinations.

### 2.2 Study

For all women baseline levels for calciotropic hormones 1,25 dihydroxyvitamin D [1,25(OH)₂D], 25 hydroxyvitamin D [25OHD] and intact parathyroid hormone (iPTH) were determined at the midpoint in the menstrual cycle. Additionally, baseline calcium levels were determined at the midpoint in the menstrual cycle. All determinations and evaluations of serum samples were performed by a single central laboratory, Nichols Institute of California.

Serum samples for the 1,25(OH)₂D assay were extracted with acetonitrile and purified by Sep-pak C-18 and Sep-Pak silica columns. The purified 1,25(OH)₂D was assayed in a radioreceptor assay using calf thymus and ₃H-1,25(OH)₂D.

The serum samples for the 25OHD assay, like the 1,25(OH)₂D samples, were extracted with acetonitrile and purified through C-18 Sep-Pak columns. The purified 25OHD sample was assayed in a radiobinding assay using ₃H-25OHD and rat serum binding protein.

The intact parathyroid hormone assay is a two site immunoradiometric assay (IRMA). The IRMA employs two kinds of anti sera, one is specific to the C-terminal portion of the molecule and the other is specific to the N-terminal end. The assay measures only the intact hormone.

The serum samples for total calcium were assayed by atomic absorption spectrometry.

The results are shown below in Table 6. Normal values for the calciotropic hormones 1,25(OH)₂D, 250HD and intact parathyroid hormone (iPTH) and calcium are shown below in Table 7.

**TABLE 6**

| CALCIOTROPIC HORMONES IN WOMEN WITH PREMENSRUAL SYNDROME | | | | | |
|---|---|---|---|---|---|
| Patient | Cycle Day | 250HD ng/ml | T.calcium mg/dl | iPTH pg/ml | 1.25(OH)₂D pg/ml |
| 001 | 14 | 18 | 9.6 | 71 | 46 |
| 002 | 12 | 17 | 9.6 | 46 | 60 |
| 003 | 15 | 20 | 8.7 | 70 | 38 |
| 004 | 13 | 24 | 9.5 | 25 | 51 |
| 005 | 12 | 17 | 9.1 | 68 | <5 |
| 006 | 15 | 16 | 9.6 | 49 | 52 |
| 007 | 15 | 16 | 8.8 | 30 | 69 |
| 008 | 13 | 17 | 8.9 | 86 | 75 |
| 009 | 17 | 27 | 9.3 | 61 | 57 |
| 010 | 16 | 24 | 8.9 | 60 | 36 |
| 011 | 13 | 33 | 9.4 | 54 | 46 |
| 012 | 14 | 25 | 9.4 | 50 | 46 |
| 013 | 14 | 19 | 9.4 | 65 | 44 |
| 014 | 15 | 27 | 9.8 | 66 | 50 |
| 015 | 13 | 21 | 9.3 | 47 | 84 |
| 016 | 13 | 27 | 10.0 | 26 | 40 |
| 017 | 15 | 23 | 9.0 | 21 | 21 |
| 018 | 14 | 27 | 9.6 | 31 | 51 |
| 019 | 14 | 35 | 9.3 | 39 | 63 |
| 020 | 14 | 24 | 9.3 | 21 | 54 |
| 021 | 15 | 21 | 8.9 | 48 | 15 |
| 022 | 15 | 21 | 9.0 | 32 | 32 |
| Cycle day refers to the day of the menstrual cycle when the serum sample was drawn. T.calcium refers to total calcium. | | | | | |

**TABLE 7**

| NORMAL CALCIOTROPIC HORMONE VALUES AS DETERMINED BY LABORATORY | | | | |
|---|---|---|---|---|
| | 250HD ng/ml | T.calcium mg/dl | iPTH pg/ml | 1.25(OH)₂D pg/ml |
| Normal Values | 9-52 | 8.8-10.4 | 10-65 | 15-60 |

### 2.3 Discussion of Lab Results

Only one patient was determined to be hypocalcemic. Five women were determined to have elevated iPTH determinations, while five were determined to have abnormal 1,25(OH)₂D levels with four elevated and one undetectable. All were determined to have normal 25OHD levels. Thus, a total of ten women were determined to have abnormally elevated iPTH or 1,25(OH)₂D determinations when these measurements were drawn at the midpoint of the menstrual cycle. It has been mentioned by Nordin et al. in an article entitled "Osteoporosis and Osteomalacia" in Clin. Endocrinal Metab., 1980; 9; 177-205 that a raised iPTH level might indicate a vitamin D deficiency. Five women were determined to have elevated iPTH levels and might be considered vitamin D deficient. However, elevated iPTH is a necessary but not a sufficient condition to absolutely diagnose a vitamin D deficiency.

### 2.4 Treatment

Each woman was instructed to take daily supplementation of 600 to 2000 IU per day with vitamin D₂ or D₃ and 1200 mg to 1500 mg per day of elemental calcium.

### 2.5 Results

Daily supplementation with vitamin D in doses of 600 to 2000 IU per day and with elemental calcium in doses of 1200 mg to 1500 mg per day resulted in a significant relief of PMS symptomatology. Within months this therapy resulted in an elevation of the 25OHD level above 30-40 ng/ml, and for those women with abnormal calciotropic values as defined by the laboratory, such values were corrected to within normal determinations. To prevent recurrence each was instructed to continue lifetime vitamin D and calcium supplementation.

### EXAMPLE 3

### Case Studies Applying Applicant's Research Finding

### 3.1 Patient X

Patient X is a 47 year old female with a 20 year history of PMS. Her major symptoms included severe irritability, mood swings, breast swelling and tenderness, and menstrual cramps. Vascular headaches, specifically common migraines (or migraines without aura) frequently interfered with her functional well being during both the premenstrual and menstrual phases of her menstrual cycle. She occasionally suffered with classic migraines (or migraines with aura) at least 4 to 5 times a year. Her common migraines were characterized by a pulsating quality of severe intensity lasting 2-3 days, associated with photophobia, nausea, occasional vomiting, and exacerbated by routine physical activity. These migraines were temporally related to the onset of her menstrual period and were always associated with PMS symptomatology. Her past medical history was significant for mild hypertension, polycystic kidney disease, mitral valve prolapse with mitral regurgitation, recurrent vaginitis, and amenorrhea 22 years ago. She had a very strong family history of breast cancer with a mother, aunt and sister all diagnosed with cancer. She is at major risk for the development of breast cancer with such a strong family history of breast cancer, a personal history of cyclical mastopathy, and a residence in the New England region. She requires an annual mammogram and breast examination for cancer screening.

Calciotropic hormone levels in this patient:
- 4/92:: total calcium 8.5 mg/dl (8.6-10.1) iPTH - 8.5 pmol/L (1.0-6.8) 25OHD - 14 mcg/L (10-80) 1,25(OH)₂D - 30.3 ng/L (18.0-62.0)
- 7/92:: total calcium 9.10 mg/dl (8.6-10.1)
- 11/92:: total calcium 9.00 mg/dl (8.6-10.1) iPTH - 7.3 pmol/L (1.0-6.8) 25OHD - 30.3 mcg/L (10.0-80.0) 1,25(OH)₂D - 50.1 ng/L (18.0-62.0)
- 2/93:: total calcium 9.70 mg/dl (8.6-10.1) iPTH - 4.30 pmol/L (1.0-6.8) 25OHD - 34.5 mcg/L (10-80)

She was diagnosed with PMS by history, by prospective charting of symptoms and by a luteal to follicular phase ratio greater than 150%. Laboratory results confirmed hypocalcemia with a secondary hyperparathyroidism and a normal 25OHD. In 4/92, she was treated with elemental calcium in the dose of 1200 mg/day and continued on her daily multivitamins (which included a low dose of elemental calcium and the RDA for vitamin D). Over the next 2 months, this resulted in complete correction of her hypocalcemia, but only partial relief of her premenstrual irritability and menstrual cramps. She was then prescribed 400 additional IU of cholecalciferol, while elemental calcium was increased to 1500 mg per day. Her vascular headaches persisted, and she still complained of nocturnal menstrual cramps. In 11/92, her total calcium was normal, her iPTH was elevated and her 25OHD remained normal as defined by the laboratory. She was prescribed 1000 IU of cholecalciferol per day and maintained on 1500 mg of elemental calcium per day in addition to her daily multivitamin (total vitamin D intake therefore amounted to 1200 IV). On this regimen, her iPTH normalized, her 25OHD increased to 34.5 mcg/L and her symptoms and migraines resolved. In addition, her blood pressure normalized. By recommending appropriate doses of vitamin D and calcium, and maintaining the 25OHD level above 35.0 mcg/L with semiannual determinations, symptomatology was prevented.

### 3.2 Patient Y

Y is a 47 year old female with a history of Rheumatic fever, mild hypertension and a 30 year history of PMS. She presented with severe premenstrual and menstrual symptomatology occurring 10 to 14 days prior to the onset of her menstrual period. Her symptoms consisted of anxiety, extreme nervousness, breast tenderness and fullness, abdominal bloating, body aches, lack of energy, vascular headaches, and severe menstrual cramps. Her symptoms were of such severity that her co-workers at her job ostracized her, and criticized her monthly abnormal behavior. With prospective charting of the daily symptoms described in Example 1.1 (less insomnia) over two menstrual cycles, PMS was confirmed. Her luteal mean score was 48 (the maximum achievable score). Baseline total calcium was 9.9 ng/ml (8.8-10.4), 25OHD was 24 ng/ml (9-52) and iPTH was 54 pg/ml (10-65). Laboratory determinations showed that she had a serum calcium that was normal as defined by the laboratory vitamin D level that was normal as defined by the laboratory and iPTH that was normal as defined by the laboratory. Prescribed daily treatment with 1200 mg of elemental calcium and 800 IU of cholecalciferol completely resolved her headaches, abdominal cramps, irritability, lethargy, breast tenderness/fullness, and behavioral changes.

The present invention is not to be limited in scope by the embodiments disclosed in the examples which are intended as illustrations of aspects of the invention. Any methods which are functionally equivalent are within the scope of this invention. Indeed, various modifications of the invention in addition to those shown and described herein will become apparent to those skilled in the art from the foregoing description. Such modifications are intended to fall within the scope of the appended claims.

## Claims

1. Use of an effective dose of vitamin D in the manufacture of a medicament for significantly reducing the symptoms of premenstrual syndrome.

2. Use according to claim 1 in which the vitamin D is selected from the group consisting of vitamin D2 ergocalciferol, vitamin D3 (cholecalciferol), and calcifediol [25OHD].

3. Use according to claim 2 in which the medicament is intended for oral administration and the oral dose of vitamin D2 is between 200 to 4000 IU per day.

4. Use according to claim 2 in which the medicament is intended for oral administration and the oral dose of vitamin D2 is between 50,000 to 200,000 IU weekly for 2-3 months.

5. Use according to claim 2 in which the medicament is intended for oral administration and the oral dose of calcifediol is between 800 to 8000 IU (20 to 200 ug) daily.

6. Use according to claim 1 in which the medicament is administered intramuscularly as vitamin D2 500,000 IU or 12.5 mg in an oil base every 2-3 months.

7. Use of a combination of calcium and vitamin D in the manufacture of a medicament for at least reducing the symptoms associated with premenstrual syndrome symptomatology.

8. Use according to claim 7 wherein the calcium is in the form of calcium carbonate.

9. Use according to claim 7 wherein the vitamin D is in the form of ergocalciferol, cholecalciferol or calcidiol.

10. Use according to claim 7 wherein the medicament is administered orally in the form of a tablet in a single daily dose.

11. Use according to claim 7 wherein the calcium is administered in the range of from about 1000 mg to about 2000 mg per day.

12. Use according to claim 11 wherein the vitamin D is administered in the range of from about 400 to about 2000 IU per day.

13. Use according to any one of claims 7 to 12 wherein the medicament is in the form of a tablet which is administered orally in a single daily dose.

14. Use according to any one of claims 7 to 13 wherein the calcium is administered in an amount of about 1200 mg per day and vitamin D in an amount of about 1000 IU per day.

15. Use according to any one of claims 7 to 14 wherein the vitamin D is administered in an amount effective to elevate the individual's 25 hydroxyvitamin D level to a level greater than 30-40 ng/ml.

16. Use according to any one of claims 7 to 15 for at least reducing vascular headaches.

17. Use according to claim 7 wherein the combination of calcium and vitamin D is in a 1:1 ratio.

18. Use of an effective dose of vitamin D in the manufacture of a medicament for significantly reducing the symptoms of migraine headache.

19. Use of a combination of calcium and vitamin D in the manufacture of a medicament for reducing the symptoms associated with migraine headache.

## Patentansprüche

1. Verwendung einer wirksamen Dosis an Vitamin D bei der Herstellung eines Medikaments zur erheblichen Verringerung der Symptome des prämenstruellen Syndroms.

2. Verwendung gemäß Anspruch 1, wobei das Vitamin D aus der Gruppe bestehend aus Vitamin D2 Calciferol, Vitamin D3 (Cholecalciferol) und Calcifediol [25OHD] ausgewählt ist.

3. Verwendung gemäß Anspruch 2, wobei das Medikament zur oralen Verabreichung bestimmt ist und die orale Dosis Vitamin D2 zwischen 200 und 4000 I.E. (IU) täglich liegt.

4. Verwendung gemäß Anspruch 2, wobei das Medikament zur oralen Verabreichung bestimmt ist und die orale Dosis Vitamin D2 zwischen 50.000 und 200.000 I.E. (IU) wöchentlich über 2 - 3 Monate liegt.

5. Verwendung gemäß Anspruch 2, wobei das Medikament zur oralen Verabreichung bestimmt ist und die orale Dosis Calcifediol zwischen 800 und 8000 I.E. (IU) (20 bis 200 ug) täglich liegt.

6. Verwendung gemäß Anspruch 1, wobei das Medikament intramuskulär als Vitamin D2 500.000 I.E. (IU) oder 12,5 mg in einer Ölbasis alle 2 - 3 Monate verabreicht wird.

7. Verwendung einer Kombination aus Calcium und Vitamin D bei der Herstellung eines Medikaments, um die Symptome in Verbindung mit der Symptomatologie des prämenstruellen Syndroms zumindest zu verringern.

8. Verwendung gemäß Anspruch 7, wobei das Calcium in Form von Calciumcarbonat vorliegt.

9. Verwendung gemäß Anspruch 7, wobei das Vitamin D in Form von Calciferol, Cholecalciferol oder Calcidiol vorliegt.

10. Verwendung gemäß Anspruch 7, wobei das Medikament oral in form einer Tablette in einer einzigen täglichen Dosis verabreicht wird.

11. Verwendung gemäß Anspruch 7, wobei das Calcium im Bereich von etwa 1000 mg bis etwa 2000 mg täglich verabreicht wird.

12. Verwendung gemäß Anspruch 11, wobei das Vitamin D im Bereich von etwa 400 bis etwa 2000 I.E. (IU) pro Tag verabreicht wird.

13. Verwendung gemäß einem der Ansprüche 7 bis 12, wobei das Medikament in Form einer Tablette ist, welche in einer einzigen täglichen Dosis verabreicht wird.

14. Verwendung gemaß einem der Ansprüche 7 bis 13, wobei das Calcium in einer Menge von etwa 1200 mg pro Tag und Vitamin D in einer Menge von etwa 1000 I.E. (IU) pro Tag verabreicht wird.

15. Verwendung gemäß einem der Ansprüche 7 bis 14, wobei das Vitamin D in einer Menge verabreicht wird, welche zur Erhöhung des 25 Hydroxyvitamin D-Werts des Individuums auf einen Wert von mehr als 30 - 40 ng/ml zu erhöhen.

16. Verwendung gemäß einem der Ansprüche 7 bis 15, um vaskuläre Kopfschmerzen zumindest zu verringern.

17. Verwendung gemäß Anspruch 7, wobei die Kombination aus Calcium und Vitamin D im Verhältnis von 1:1 steht.

18. Verwendung einer wirksamen Dosis an Vitamin D bei der Herstellung eines Medikaments zur erheblichen Verringerung der Symptome von Migränekopfschmerzen.

19. Verwendung einer Kombination aus Calcium und Vitamin D bei der Herstellung eines Medikaments zur Verringerung der Symptome in Verbindung mit Migränekopfschmerzen.

## Revendications

1. Utilisation d'une dose efficace de vitamine D dans la fabrication d'un médicament destiné à réduire de façon significative les symptômes du syndrome prémenstruel.

2. Utilisation suivant la revendication 1, dans laquelle la vitamine D est choisie dans le groupe consistant en vitamine D2, ergocalciférol, vitamine D3 (cholécalciférol) et calcifédiol (25OHD).

3. Utilisation suivant la revendication 2, dans laquelle le médicament est prévu pour l'administration orale et la dose orale de vitamine D2 est comprise entre 200 et 4 4000 UI par jour.

4. Utilisation suivant la revendication 2, dans laquelle le médicament est prévu pour l'administration orale et la dose orale de vitamine D2 est comprise entre 50 000 et 200 000 UI par semaine pendant 2 à 3 mois.

5. Utilisation suivant la revendication 2, dans laquelle le médicament est prévu pour l'administration orale et la dose orale de calcifé diol est comprise entre 800 et 8 000 UI (20 à 200 µg) par jour.

6. Utilisation suivant la revendication 1, dans laquelle le médicament est administré par voie intramusculaire à raison de 500 000 UI ou 12,5 mg de vitamine D2 dans une base d'huile tous les 2 à 3 mois.

7. Utilisation d'une combinaison de calcium et de vitamine D dans la fabrication d'un médicament destiné à réduire au moins les symptômes associés à la symptomatologie du syndrome prémenstruel.

8. Utilisation suivant la revendication 7, dans laquelle le calcium est sous la forme de carbonate de calcium.

9. Utilisation suivant la revendication 7, dans laquelle la vitamine D est sous la forme d'ergocalciférol, de cholécalciférol ou de calcidiol.

10. Utilisation suivant la revendication 7, dans laquelle le médicament est administré par voie orale sous la forme d'un comprimé en une dose quotidienne unique.

11. Utilisation suivant la revendication 7, dans laquelle le calcium est administré en une quantité comprise dans la gamme d'environ 1000 mg à environ 2000 mg par jour.

12. Utilisation suivant la revendication 11, dans laquelle la vitamine D est administrée en une quantité comprise dans la gamme d'environ 400 à environ 2000 UI par jour.

13. Utilisation suivant l'une quelconque des revendications 7 à 12, dans laquelle le médicament est sous la forme d'un comprimé qui est administré par voie orale en une dose quotidienne unique.

14. Utilisation suivant l'une quelconque des revendications 7 à 13, dans laquelle le calcium est administré en une quantité d'environ 1200 mg par jour et la vitamine D en une quantité d'environ 1000 UI par jour.

15. Utilisation suivant l'une quelconque des revendications 7 à 14, dans laquelle la vitamine D est administrée en une quantité efficace pour élever le taux de 25-hydroxyvitamine D d'un individu jusqu'à un niveau supérieur à 30-40 ng/ml.

16. Utilisation suivant l'une quelconque des revendications 7 à 15, pour réduire au moins les maux de tète d'origine vasculaire.

17. Utilisation suivant la revendication 7, dans laquelle la combinaison de calcium et de vitamine D est selon un rapport 1:1.

18. Utilisation d'une dose efficace de vitamine dans la fabrication d'un médicament destiné à réduire de façon significative les symptômes des maux de tête migraineux.

19. Utilisation d'une combinaison de calcium et de vitamine D dans la fabrication d'un médicament destiné à réduire les symptômes associés aux maux de tête migraineux.
